(19) Europäisches Patentamt
European Patent Office
Office européen des brevets

(11) Publication number: **0 458 892 B1**

(12) **EUROPEAN PATENT SPECIFICATION**

(45) Date of publication of patent specification: **15.06.94**

(51) Int. Cl.⁵: **A61K 31/425**, //C07D277/22

(21) Application number: **90903980.2**

(22) Date of filing: **13.02.90**

(86) International application number:
**PCT/SE90/00093**

(87) International publication number:
**WO 90/09174 (23.08.90 90/20)**

# (54) USE OF CHLORMETHIAZOLE IN THE MANUFACTURE OF A MEDICAMENT FOR THE PREVENTION AND/OR TREATMENT OF NEURODEGENERATION.

(30) Priority: **17.02.89 SE 8900564**

(43) Date of publication of application:
**04.12.91 Bulletin 91/49**

(45) Publication of the grant of the patent:
**15.06.94 Bulletin 94/24**

(84) Designated Contracting States:
**AT BE CH DE DK ES FR GB GR IT LI LU NL SE**

(56) References cited:
**SE-C- 196 140**

**Annals of Neurology, Vol. 24, No. 6, December 1988, pp. 699-707: "Biological differences between Ischemia, Hypoglycemia and Epilepsy" by Auer et al.**

**Acta psy. scand. Suppl. 329, vol. 73, 1986 N T Gurusinghe et al. *see page 189 - page 193***

(73) Proprietor: **AKTIEBOLAGET ASTRA**

**S-151 85 Södertälje(SE)**

(72) Inventor: **BOAR, Robin, Bernard**
**25 Meadow Way**
**Letchworth**
**Hertfordshire SG6 3JB(GB)**
Inventor: **CROSS, Alan, John**
**54 Birchwood Road**
**West Byfleet**
**Surrey KT14 6DW(GB)**
Inventor: **GREEN, Alfred, Richard**
**22 Laurel Drive**
**Southmoor**
**Abingdon Oxon OX13 5DG(GB)**

(74) Representative: **Hjertman, Ivan T. et al**
**AB ASTRA**
**Patent and Trade Mark Department**
**S-151 85 Södertälje (SE)**

**EP 0 458 892 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Acta psych. scand. Suppl. 329, vol. 73, 1986 S A Ather et al. *see page 81-page 91, see esp. pages 81 and 88*

Archives of disease in childhood, vol. 57, 1982, T V Stanley, *see page 242-243

British Medical Jouranl, vol. 280, Febr. 1980, L Low eta l. *see page 484

Dialog Inform. Serv., File 155, Medline 85271668, Dialog accession no. 0565668, Nouailhat F et al.

-acta anaesth. scand., vol. 23, 1979 C Carlsson et al. *see page 259-266, see esp. pages 259-260*

Anaesthesist, vol. 22, 1973, I Piclmayr et al. *see page 496-500, see esp. page 496*

ournal of the Royal College of General Practitioners, vol. 32, Dec. 1982 M G Mead, *see page 763-765*

## Description

This invention relates to a new medical use for a heterocyclic compound and pharmaceutical compositions containing it. In particular, it relates to the use of 5-(2-chloroethyl)-4-methylthiazole and the pharmaceutically acceptable salts and solvates thereof, in the manufacture of a medicament for the prevention and/or treatment of neurodegeneration in pathological conditions such as stroke, cerebral ischaemia, hypoxia, epilepsy and neurodegenerative diseases such as Alzheimer's disease, multi-infarct dementia and Huntington's disease. This thiazole compound with which the present invention is concerned has the following structural formula

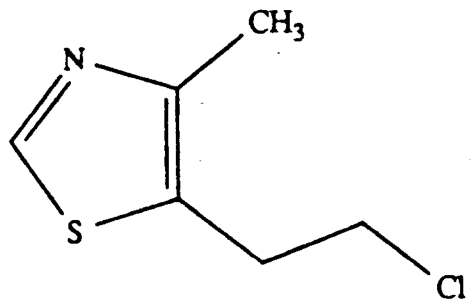

and is known alternatively as chlormethiazole, chlomethiazole or chlorethiazole.

## Background to the Invention

Preparations containing chlormethiazole, particularly in the form of its acid addition salts, especially, as disclosed in GB 847,520, the acid addition salt with ethanedisulphonic acid, are known to possess valuable therapeutic properties. The general pharmacology and therapeutic applications of chlormethiazole have been extensively reviewed in a recent publication ( Acta Psychiatr. Scand., Suppl. 329, 73, 1986). Thus, for example, chlormethiazole possesses sedative and hypnotic properties and is used clinically as a hypnotic in the elderly, particularly in the management of psychogeriatric patients. Chlormethiazole also possesses anticonvulsant properties and is used clinically for the treatment of different types of convulsive states, such as, for example, status epilepticus and pre-eclampsia. Chlormethiazole is also used clinically for the treatment of ethanol (alcohol) withdrawal states including delirium tremens.

The present invention can be distinguished from the above prior art in that it is concerned with a new medical use which is unexpected and which can be clearly distinguished from the applications in the above described disorders. Usefulness in any or all of the above clinical disorders would not suggest or in any way make obvious the use of the compounds which are the subject of the present invention in the prevention and/or treatment of neurodegeneration.

There are a few contradictory reports on the effects of chlormethiazole on cerebral circulation and metabolism. I. Pichlayr and co-workers ( Anaesthesist, 1973, 22, 496-500) found that i.v. infusion of chlormethiazole into dogs caused an initial increase in cerebral blood flow, followed by a decrease in both cerebral blood flow and oxygen consumption rate, a finding which they interpreted as indicating that chlormethiazole possibly offers a degree of protection to cerebral functions. Others (J. Pogady, M. Ruscak and J. Orlicky, Activ. Nerv. Sup. (Prague), 1972, 14, 87) have presented results which are interpreted as possibly indicating a deleterious effect of chlormethiazole in situations associated with impaired brain circulation. Significantly, in the most recent study ( Acta Anaesth. Scand., 1979, 23, 259-266) Carlsson and Rehncrona were unable to demonstrate either a protective or a detrimental effect of chlormethiazole when it was administered during reversible, incomplete ischaemia in the rat. None of the above studies considered an action of chlormethiazole on neurodegeneration following pathological insult.

The present invention provides the use of 5-(2-chloroethyl)-4-methyl-thiazole, or a pharmaceutically acceptable salt or solvate thereof in the manufacture of a medicament for the prevention and/or treatment of neurodegeneration in the above mentioned pathological conditions. Thus the new use of chlormethiazole that is the subject of the present invention could not in any way be expected from the above prior art.

Other compounds have been reported to be useful in the prevention and/or treatment of neurodegeneration (see, for example, EP 230 370 and EP 273 309), but there is no drug that is presently accepted as a standard for this use. None of the other compounds thus described would suggest the use of the compound of the present invention for the prevention and/or treatment of neurodegeneration.

## Detailed Description of the Invention

Different aspects of the present invention are:

the use of 5-(2-chloroethyl)-4-methylthiazole, or a pharmaceutically acceptable salt or solvate thereof, in the manufacture of a medicament for the prevention and/or treatment of neurodegeneration, especially in connection with conditions such as stroke, cerebral ischaemia, hypoxia, epilepsy and in neurodegenerative diseases such as Alzheimer's disease, multi-infarct dementia and Huntington's disease;

Pharmaceutically acceptable salts include, besides the said salt with ethanedisulphonic acid, salts with methane polysulphonic acids, ethane monosulphonic acids and ethane polysulphonic acids. The salt with ethanedisulphonic acid is the preferred salt.

The clinically most important of the new fields of use are considered to be the prevention and/or treatment of neurodegeneration in connection with stroke, cerebral ischaemia, multi-infarct dementia and hypoxia.

The effectiveness of 5-(2-chloroethyl)-4-methylthiazole for use according to the present invention in the prevention and/or treatment of neurodegeneration may be demonstrated by the ability to decrease damage to the CA1/CA2 hippocampal pyramidal neurones in gerbils following ischaemia induced by a period of occlusion of the carotid arteries followed by reperfusion. The detailed mechanisms that underlie ischaemia-induced degeneration of hippocampal neurones have yet to be clarified, but the above mentioned gerbil test system has been widely used as a predictive model of neuroprotective activity (see, for example, B.J. Alps, C. Calder, W.K. Hass and A.D. Wilson, Brit. J. Pharmacol., 1988, 93, 877-883; R. Gill, A.C. Foster and G.N. Woodruff, J. Neuroscience, 1987, 7, 3343-3349).

It is a particular feature that the new use of the present invention is effective in the manufacture of a medicament for the preventing and/or treating neurodegeneration not only when administered prior to the ischaemic insult, but also when administered solely after the ischaemic insult, even several hours after the ischaemic insult. It is to be expected that the efficacy when administered post-ischaemia is of particular relevance to the likely clinical utility.

The administration of medicaments prepared according to the use of this invention may conveniently be oral, rectal, or parenteral at a dosage level of, for example, about 1 to 3000 mg/kg, preferably about 10 to 1000 mg/kg and especially about 25 to 250 mg/kg and may be administered on a regimen of 1 to 4 times per day. The dose will depend on the route of administration, a particularly preferred route being by intravenous infusion of an aqueous solution containing chlormethiazole ethanedisulphonate, for example, an aqueous solution containing chlormethiazole ethanedisulphonate 8 mg/ml. It will be appreciated that the severity of the disease, the age of the patient and other factors normally considered by the attending physician will influence the individual regimen and dosage most appropriate for a particular patient.

The pharmaceutical formulations comprising the compound used according to this invention may conveniently be tablets, pills, capsules, powders or granules for oral administration; sterile parenteral solutions or suspensions for parenteral administration; or as suppositories for rectal administration.

## Neuroprotection Studies

Ischaemia was induced in gerbils by occlusion of the carotid arteries following the accepted general procedure as described in, for example, EP 230 370 and R. Gill, A.C. Foster and G.N. Woodruff, J. Neuroscience, 1987, 7, 3343-3349.

Typical procedures and results are exemplified as follows:

### *a) Administration of the Test Compound Prior to Induction of Ischaemia.*

Ischaemia was induced in gerbils by 10 minute occlusion of both carotid arteries. Restoration of blood flow after occlusion was checked visually and the animals were allowed to survive for 4 days. The extent of neuronal degeneration in the hippocampus was then assessed. The test compounds were administered (i.p.) as a single dose 30 minutes prior to occlusion. Typical results are shown in Table 1. As is seen in Table 1, chlormethiazole was effective in reducing the damage to the CA1/CA2 hippocampal neurones.

Table 1

| Test compound | Dose | Number of Animals | % Damage to CA1/CA2 Hippocampal Neurones |
|---|---|---|---|
| None | -- | 10 | 94.9 ± 2.0 |
| Chlormethiazole ethanedisulphonate | 100mg/kg | 7 | < 5 |
| Chlormethiazole ethanedisulphonate | 50mg/kg | 8 | 37.6 ± 13.4 |
| **Prior Art Compounds** | | | |
| MK 801[a] | 3mg/kg | 6 | < 5 |
| Ifenprodil[b] | 4mg/kg | 7 | 62 ± 8.0 |

References to Prior Art Compounds

a: See, for example, EP 230 370

b: See, for example, E.T. MacKenzie, B. Gotti, J-P. Nowicki & A.R. Young (1984) in 'Neurotransmitters and the Cerebral Circulation'. Raven Press, New York, pp219-243.

*b) Administration of the Test Compound Following Induction of Ischaemia.*

The general procedure described in (a) above was followed except that the test compound was administered (i.p.) as a single dose at the stated time following the end of the occlusion. No administration

was made prior to the occlusion. Typical results are shown in Table 2. As is seen in Table 2, chlormethiazole was effective in reducing the damage to the CA1/CA2 hippocampal neurones when administered after the ischaemic insult.

Table 2

| Test compound | Dose | Time of Dosing after Induction of Ischaemia | Number of Animals | % Damage to CA1/CA2 Hippocampal Neurones |
| --- | --- | --- | --- | --- |
| None | -- | -- | 9 | 80.4 ± 7.9 |
| Chlormethiazole ethanedisulphonate | 100mg/kg | 30 minutes | 8 | 12.8 ± 10.4 |
| Chlormethiazole ethanedisulphonate | 50mg/kg | 30 minutes | 8 | 69.4 ± 6.9 |
| Chlormethiazole ethanedisulphonate | 100mg/kg | 1 hour | 9 | 14.3 ± 8.7 |
| Chlormethiazole ethanedisulphonate | 100mg/kg | 2 hours | 8 | 7.25 ± 4.8 |
| Chlormethiazole ethanedisulphonate | 100mg/kg | 6 hours | 10 | 45.6 ± 14 |

## Claims

1. The use of 5-(2-chloroethyl)-4-methylthiazole, or a pharmaceutically acceptable salt or solvate thereof in the manufacture of a medicament for the prevention or treatment of neurodegeneration.

2. The use according to claim 1 in the manufacture of a medicament for the prevention or treatment of neurodegeneration in connection with stroke.

3. The use according to claim 1 in the manufacture of a medicament for the prevention or treatment of neurodegeneration in connection with cerebral ischaemia.

4. The use according to claim 1 in the manufacture of a medicament for the prevention or treatment of neurodegeneration in connection with hypoxia.

5. The use according to claim 1 in the manufacture of a medicament for the prevention or treatment of neurodegeneration in connection with multi-infarct dementia.

## Patentansprüche

1. Verwendung von 5-(2-Chlorethyl)-4-methylthiazol, oder eines pharmazeutisch annehmbaren Salzes oder Solvates hievon, bei der Herstellung eines Medikamentes zur Prävention oder Behandlung einer Neurodegeneration.

2. Verwendung nach Anspruch 1, bei der Herstellung eines Medikamentes zur Prävention oder Behandlung einer Neurodegeneration in Verbindung mit einem Schlaganfall.

3. Verwendung nach Anspruch 1, bei der Herstellung eines Medikamentes zur Prävention oder Behandlung einer Neurodegeneration in Verbindung mit zerebraler Ischämie.

4. Verwendung nach Anspruch 1, bei der Herstellung eines Medikamentes zur Prävention oder Behandlung einer Neurodegeneration in Verbindung mit Hypoxie.

5. Verwendung nach Anspruch 1, bei der Herstellung eines Medikamentes zur Prävention oder Behandlung einer Neurodegeneration in Verbindung mit Multiinfarkt-Demenz.

## Revendications

1. Utilisation du 5-(2-chloroéthyl)-4-méthylthiazole ou d'un sel pharmaceutiquement acceptable ou d'un produit de solvatation ou solvat de ce composé, dans la fabrication d'un médicament pour le traitement préventif ou curatif d'une neurodégénérescence.

2. Utilisation selon la revendication 1 dans la fabrication d'un médicament pour le traitement préventif ou curatif d'une neurodégénérescence en liaison avec une attaque.

3. Utilisation selon la revendication 1 dans la fabrication d'un médicament pour le traitement préventif ou curatif d'une neurodégénérescence en liaison avec une ischémie cérébrale.

4. Utilisation selon la revendication 1 dans la fabrication d'un médicament pour le traitement préventif ou curatif d'une neurodégénérescence en liaison avec de l'hypoxie.

5. Utilisation selon la revendication 1 dans la fabrication d'un médicament pour le traitement préventif ou curatif d'une neurodégénérescence en liaison avec de la démence associée à des infarctus multiples.